# EUROPEAN PATENT SPECIFICATION

(11) **EP 0 405 993 B1**
(45) Date of publication and mention of the grant of the patent: **06.12.1995**
(21) Application number: 90307113.2
(22) Date of filing: 28.06.1990
(51) Int. Cl.: A61L 15/60, A61L 15/28

(54) **Superabsorbent wound dressing**
Superabsorbierender Wundverband
Pansemet superabsorbant

(30) Priority: 28.06.1989 US 372478; 28.06.1989 US 372477
(43) Date of publication of application: 02.01.1991
(73) Proprietor: JAMES RIVER CORPORATION OF VIRGINIA, Richmond, VA 23217 (US)
(72) Inventor: Hollenberg, David H., Neenah WI 54956 (US); Van Luu, Phuong, 68240 Kaysersberg (FR)
(74) Representative: Cropp, John Anthony David

(56) References cited:
- EP-A- 0 019 371
- WO-A-83/00289
- WO-A-87/03208
- US-A- 4 090 013

## Description

The present invention relates to wound dressings. More particularly, the invention relates to wound dressings formed from an improved water-swellable, water-insoluble superabsorbent polymer. The invention also provides superabsorbent polymers having increased absorbancy in the presence of dissolved salts and suitable for use in the dressings and a method of forming superabsorbents.

Superabsorbents are generally composed of high molecular weight hydrophilic polymers which can be crosslinked to provide water-insoluble but water-swellable materials. Depending on the type of polymer and the type and extent of crosslinking, these polymers may be capable of absorbing many times their own weight in fluid. The types of polymers useful for producing super-absorbents in this manner have been described generally in United States Patent No. 4,090,013. However, it is known that such water-insoluble/water-swellable compositions lose some of their absorbent capacity when the fluid to be absorbed is blood or other serous exudates caused by wounds or burns.

Upon contact with physiological fluids of high ionic strength, it is known that polymer absorbents tend to form a barrier around the fluid which markedly reduces the penetration of the fluid by the absorbent product. It is also known from the prior art that addition of certain polyhydroxylated substances such as inorganic or organic salts or oligosaccharides (simple sugars) can increase the absorbency of the superabsorbent by accelerating the capillary flow of the bodily fluid through the mass of the particulate absorbent.

Examples of patents in which polyhydroxylated materials are added to a superabsorbent include those to Chmelir et al., U.S. 4,693,713, and to Lerailler, et al., WO 87/03208. However, in the composition products produced according to these patents, the polyhydroxylated component present in the absorbent can be dissolved into the bodily fluids to which the absorbent is applied with the result that the absorbent is less effective as a dressing and is, moreover, a possible contaminant.

Therefore, there is a need for a flexible superabsorbent which will have bacteriostatic properties and which will not dissolve upon contact with bodily fluids.

It has now been found that the above and related objects of the present invention are obtained by the polyhydroxylate/polymer crosslinked compositions of the present invention which meet the need of the prior art and are especially suitable as wound dressings because of their absorbent capabilities and their flexibility.

Thus, according to the present invention, there is provided a water-swellable, water insoluble superabsorbent composition comprising an alkali soluble polyelectrolyte admixed with a polyhydroxylated material which is crosslinked by means of a polyvalent metal ion crosslinker and neutralized by addition of a base to provide an absorbent polymer wherein the polyhydroxylated material is immobilized in said polymer.

The superabsorbent composition may contain up to 90 % by weight of alkali soluble polyelectrolyte.

The present invention also provides a wound dressing formed by drying a water-swellable, water-insoluble absorbent composition comprising an alkali soluble polyelectrolyte in solution with a polyhydroxylated material to which is added a polyvalent metal ion crosslinker.

In accordance with one embodiment of the present invention, a synthetic carboxylate of sulfonated polymer, as for example, polyacryiic acid or partially hydrolyzed polyacrylamide, or other alkali soluble polyelectrolyte is dissolved in solution with a polyhydroxylated material such as a simple saccharide. A polyvalent metal ion crosslinker e.g. a quadrivalent or combination quadrivalent/trivalent metal ion is then added to the solution, resulting in the immobilization of the polyhydroxylate material into the water-swellable, water-insoluble superabsorbent composition.

Incorporation of polyhydroxylated materials such as saccharides into the metal ion-crosslinked polymer forming the absorbent increases the absorbency of the material and enhances its response to physiologic fluids such as blood or secretions from wounds or burns. Further, the high concentration of polyhydroxylated material such as saccharide in these products may provide anti-bacterial and anti-microbial properties to dressings formed therefrom. The thin film, thick plaque or cellular foam embodiments of the polymers used in absorbents containing the invention are especially useful as wound dressings because they will not be compromised by limitations of thickness, flexibility, density, absorbency or most importantly, of solubility. Because the components of the polymers and the added polyhydroxylated material such as saccharide are innocuous in and of themselves and are crosslinked or immobilized in the final absorbent product, the possibility of irritation or provocation of allergic response upon application to a wound is minimized. It will be understood that the metal ion crosslinker should also be pharmacologically acceptable. Further, the fact that polyhydroxylated materials such as saccharide in an amount up to 80%, e.g. 10-80 %, by weight of the product can be incorporated and immobilized into the crosslinked polymer network provides a great range of flexibility to the absorbent films, plaques and foams produced, varying from very flexible to quite stiff. Yet whatever the degree of flexibility, the structural integrity of the wound dressing is not compromised over time because the immobilized polyhydroxylated component is no longer soluble.

Finally, the incorporation of large amounts of polyhydroxylated material such as saccharide into the absorbent lowers the cost of the absorbent significantly; saccharides such as sucrose costing less than 1/10 of the cost of the polymer by unit weight while at the same time reducing the sensitivity of the absorbent material to dissolved salts in an aqueous medium.

Exemplary of the polymers useful in the present invention are the carboxylated or sulfonated polymers described in U.S. Patent No. 4.090,013. Exemplary of the polyhydroxylated materials to be used are any simple sugars, i.e., mono-, di-, or oligosaccharides such as sucrose, glucose, fructose, mannose or saccharose. Also may be mentioned simple diols and triols, soluble starches, low molecular weight polyvinyl alcohols as well as other polyhydroxylated low molecular weight polymers. The crosslinking agents are polyvalent metal cations, preferably having a valence of at least three or four, which render the crosslinked hydroxylate polymer composition insoluble yet highly swellable. Preferred metal cations include, but are not limited to, zirconium and combinations of zirconium and aluminum. Preferred sources for the metal cations are salts which include, but are not limited to, aluminum/zirconium lactate, aluminum/zirconium acetate, and ammonium zirconium carbonate.

In one preferred embodiment of the invention a solution is prepared wherein polyacrylic acid is mixed with sucrose in a ratio of at least 50%, e.g., from 50% to 80%, by weight of the sucrose. The solution is neutralized to achieve a pH of about 7 by addition of a base such as, for example, an ammonium compound such as ammonium carbonate or ammonium hydroxide or a combination thereof. The polyvalent metal ion, either zirconium or aluminum/zirconium, is added and the viscous solution or foam can be extruded into a film, foam or thick plaque. The resultant films, plaques or foams are then dried for use alone or added to a paper substrate and dried.

The present invention is a very efficient system for producing, inter alia, a wound dressing, which is a humectant; i.e., a substance that promotes retention of moisture. By absorbing physiological fluids from the wounds, the wound site is dried and any microbes or bacteria present are dessicated and die. Because the polyhydroxylated material such as saccharide is immobilized in the absorbent, it does not dissolve into the wound which would have the double effect of maintaining moisture in the wound and contaminating the wound.

The precise mechanism by which polyhydroxylated material such as saccharide is immobilized in the polymer forming the absorbent complex is not known. It could result from crosslinking of these polymer materials with themselves as mediated by the metal ions, crosslinking with the polymer via the metal ion, or a reaction with the polymer to form esters. Other mechanisms might also be responsible for immobilization.

While the use of metal ions as crosslinkers has been disclosed in the prior art, see for example U.S. Patent Number. 4,090,013, new limitations exist with regard to their applications in the present invention and/or in the presence of polyhydroxylated materials. In dealing with emulsions, the metal ion must be stable to the pH of the bulk phase and should not react or interact with the ionic or organic dispersants or stabilizers contained therein. The amounts of crosslinker added will depend not only on the type of polymer used but also on the type and amount of polyhydroxylated material used. Since the polyhydroxylated components are generally of lower molecular weight than the polymer, as the proportion of polyhydroxylated material increases the amount of added crosslinker must also increase. Although all of the metal ions previously disclosed in U.S. Patent No. 4,090,013 may be used in the practice of the present invention, the use of zirconium ions alone or in admixture with ferric, aluminum, chromic, or titanium ions has been found to be especially useful.

In all of the prior art known to the present inventors in which polyhydroxylated materials are added to polymers to form a superabsorbent wound dressing, the polyhydroxylated materials are not immobilized in the polymer of the superabsorbent material; hence, it can be dissolved by contact with bodily fluids and can leach out of the dressing and thereby contaminate the wound. An additional advantage of the present invention is that because the polyhydroxylated material is immobilized in the dressing, the resultant forms of the dressing whether they be films, plaques or foams, can be made stiff or pliable depending upon the amount of polyhydroxylated material and water added to the composition. Moreover, they can be made thick for pressure bandages or as thin as a Band-Aid, and through manipulation of the composition in a manner known in the art, the film can be made to stick to itself. For example, if placed around a finger, one end of a film will adhere to the other end of the film.

A preferred embodiment of the present invention relates to the preparation of a wound dressing wherein high concentrations of immobilized polyhydroxylated material such as sucrose are incorporated therein by addition of metal ion crosslinkers to solutions or emulsions containing polyhydroxylate and alkali soluble polyelectrolyte. Zirconium and mixtures of zirconium/aluminum ions are used as suitable cross-linkers to provide water-insoluble, water-swellable polymers containing from 10% to 80% polyhydroxylate. It is believed that the zirconium crosslinks the hydroxyl groups of the polyhydroxylate and ties it to the large crosslinked polymer thereby creating one large complexed molecule. The pH of the solution is neutralized, if necessary, by the addition of ammonium hydroxide or ammonium carbonate and the resultant mixture is dried.

The invention also relates to a water-swellable, water-insoluble superabsorbent composition comprising an alkali soluble polyelectrolyte admixed with a polyhydroxylated material which is crosslinked with a polyvalent metal ion crosslinker and neutralized by addition of a base to provide an absorbent polymer wherein the polyhydroxylated material is immoblilized in said polymer.

Although a great variety of superabsorbents based on a wide range of chemistries have been described, there are basically just two methods for producing superabsorbents. One method involves polymerization of low molecular weight precursors to provide superabsorbents directly; the second method involves the crosslinking of preformed polymers. In forming superabsorbents by either method, the control of the amount of crosslinking (crosslink density) is very important with regard to the performance of the superabsorbent.

One of the problems associated with crosslinking of preformed polymers is that typically the polymer is of high molecular weight and when these materials are dissolved in water, they form very viscous solutions even at low concentrations. In order to thoroughly mix the crosslinker with the polymer, the solids in solution must be fairly dilute, on the order of 0.5% or less. Such extensive dilution requires large tankage capacity and the expenditure of much money, energy and time to rid the final polymer product of the water necessary to make it. Therefore, there is a need to develop a process wherein the polymer materials can be mixed at higher solids concentrations, thereby reducing the amount of water needed in the process and achieving savings in energy, size of equipment, effluent control and manufacturing time.

The present invention provides a method to form crosslinked superabsorbents at relatively high solids concentrations of polymer wherein low molecular weight, essentially non-ionic, hydrophilic molecules i.e. polyhydroxylated materials can be incorporated and immobilized.

The invention further relates to a method for preparing a superabsorbent containing high concentrations of polyhydroxylated material which comprises mixing a polyhydroxylated material with a solution or emulsion of a polyelectrolyte, adding a volatile neutralizing compound and adding a polyvalent metal ion crosslinker and drying said composition.

Incorporation of polyhydroxylates, such as starches and saccharides in the composition, reduces both the cost of the superabsorbent as well as the sensitivity of the superabsorbent composition to dissolved salts in the aqueous medium. It also provides increased flexibility to the final composition. the superabsorbents thus produced may be used in the thin film, powder, in a bulk form (thick film or plaque), or as a coating or binding for paper or nonwovens and especially in wound dressings.

In the present invention, high concentrations of polymer solids can be incorporated into a superabsorbent composition by starting with an emulsion of a high molecular weight polymer, such as an alkali soluble polyelectrolyte, subsequently adding a suitable metal ion crosslinker to the bulk phase of the emulsion and then adding a volatile base to bring about in situ solubilization of the polymer. Subsequent evaporation of the water provides the superabsorbent. The advantage of this process is that the production of the superabsorbent is accomplished at a relatively high solids content.

Another advantage of the method of the present invention is that it has been found that high concentrations of relatively low molecular weight polyhydroxylated compounds can be added to a mixture of polyelectrolyte and metal ion crosslinker prior to solubilization. Addition of these simple polyhydroxylated materials to the mixture results in superabsorbents with improved performance and provides a significant reduction in the cost of the superabsorbent on an absorbency per unit weight basis. The discovery that polyhydroxylated materials can be incorporated into the final crosslinked structure is not confined to the system where one uses an emulsion of the polyelectrolyte, but has been found useful in producing superabsorbents starting with solutions of polyhydroxylated materials admixed with solutions of polyelectrolytes as well as with emulsions of polyelectrolytes.

Examples of the types of polyhydroxylated materials that may be employed have been given above. Admixture of these compounds with the polyelectrolytes should be done before the crosslinking via metal ions is accomplished. By use of the proper crosslinking ion(s), the polyhydroxylated materials will be immobilized in the final product.

Although all of the metal ions previously disclosed in U.S. Patent No. 4,090,013 may be used in the method according to the present invention, the use of zirconium ions alone or in admixture with ferric, aluminum, chromic, or titanium ions has been found to be especially useful.

A preferred embodiment of the method according to the present invention comprises (1) addition of a polyhydroxylate in a range up to 80% by weight to an alkali soluble polyelectrolyte in emulsion or solution, (2) adding a volatile neutralizing agent, if necessary, to adjust the pH of the solution to approximately 7, (3) adding metal crosslinkers having valences of three or more to the polyhydroxylate-polyelectrolyte emulsion and mixing thoroughly, and (4) drying the resultant mixture, i.e., in a convection oven, by microwave heating, by ambient evaporation or other conventional thermal processes.

In accordance with the preferred embodiment of the method of present invention, the superabsorbent of the present invention is composed of an emulsion of polyacrylic acid in water to which 50% to 80% saccharide in solution or in dry granules and a metal ion crosslinker containing zirconium are added and mixed. It is believed that the zirconium crosslinks the hydroxyl groups of the saccharide and ties it to the large crosslinked polymer thereby creating one large complexed molecule. The pH of the solution is neutralized, if necessary, by the addition of, e.g., ammonium hydroxide or ammonium carbonate or other volatile bases and the resultant mixture is dried. When starting with an emulsion of polyacrylic acid, the crosslinker can be added to the emulsion before pH adjustment.

The resultant films, plaques or foams, are then dried for use alone or added to a paper substrate and dried. These absorbent compositions can be used as wound dressings as well as films, foams or powders in absorbent products, binders or adhesives in non-wovens and composites, film or coatings in food wraps or multilayer laminants.

The methods for forming films, foams, and plaques are similar. In a preferred method saccharide is mixed with polyacrylate emulsion and before the crosslinker is added. The pH of the solution is adjusted to approximately 7 with a base such as ammonium hydroxide (or a mixture of ammonium hydroxide and saturated ammonium carbonate for foams). The solution can be cast as a film or poured into a mold and dried either in a drier, or it can be allowed to air dry. Alternatively, the polyhydroxylate e.g. sugar may be mixed with a solution of polyelectrolyte, and after adjustment of the pH of the solution to between 7 and 8, the crosslinker may be added. If the crosslinker is added when the pH is below 7, the mixture will form a gel immediately.

In the following examples, the polyelectrolytes are Acrysol® A-3 or ASE-75, solutions and/or emulsions of polyacrylic acid in water, obtained from Rohm, & Haas Company, Philadelphia, PA. The Zirtech® crosslinkers, aluminum/zirconium lactate (AAL) and aluminum/zirconium acetate (LAA) were obtained from Zirtech, Inc., Gainesville, Florida. The ammonium zirconium carbonate was obtained from Magnesium Elektron, Twickenham, U.K. Bacote 20 is a stablilized solution of zirconium ions available from Magnesium Elektron, Flemington, NJ.

The following examples demonstrate some of the many variations in compositions and procedures which can be followed.

Examples 1 to 7 illustrate the superabsorbent materials and methods of preparing superabsorbents according to the present invention. Examples 8 to 11 specifically illustrate procedures used for obtaining wound dressings.

### Example 1

A polyacrylate solution was prepared as follows: Acrysol® A-3 (100 g solution, 25% solids) was mixed with solid sodium hydroxide pellets (8.2 g) to provide a solution of - 65% neutralized polyacrylic acid. Ammonium hydroxide (10.5 g, 28% NH₃ in water) and ammonium carbonate (5.0 g) were added to this solution and completely dissolved. Portions of this solution were also used in Examples 2-3 below.

A portion (10 g of solution, - 25% solids) of the Acrysol® A-3 solution described above was mixed with maltose (3 grams). After the maltose dissolved in the polyacrylate solution, Bacote® 20 (1.0 g of solution) was added. After thorough mixing, a portion of the material was dried in a microwave oven to a light, absorbent foam.

### Example 2

A portion (10 g of solution) of the Acrysol® A-3 solution was mixed with a solution of glucose (5 g) dissolved in warm water (5 g). Bacote® 20 (1.0 g of solution) was added, and after mixing a portion of this mixture was spread on a glass plate and allowed to dry at ambient temperature. After 24 hours, a thin flexible absorbent film was obtained.

### Example 3

A portion (10 g of solution) of the Acrysol® A-3 solution was mixed with a solution of mannitol (3 g) in water (5 g). Bacote® 20 (1.0 g solution) was added, and after mixing a portion of this solution was dried in a convection oven to provide an absorbent foam.

### Example 4

Acrysol® ASE-75 (300 g, 120 g solids) was mixed with sucrose (120 g). This mixture was diluted to 2400g with water. Aluminum/zirconium lactate (AAL) (9.3 g solution) was added, then ammonium hydroxide (29% ammonia in water) was added to bring the solution to pH of 7-7.5. The thick solution could be spread into films and dried at ambient conditions to provide an absorbent film.

### Example 5

Acrysol® ASE-75 (1045 g, 418 g solids) was mixed with sucrose (324.5 g) and diluted to 3000g with water. Aluminum/zirconium lactate (AAL) (112.3 g solution), was added and after thorough mixing, this suspension of latex and sugar was mixed with an equal volume of a saturated solution of ammonium carbonate. The resulting foam could be extruded into very thick films and dried in an oven at 105°C to provide absorbent foams.

### Example 6

Acrysol® ASE-75 (5 g, 2 g solids) was mixed with Aluminum/zirconium lactate (AAL) (0.6 g solution). Sucrose (1.0 g) was dissolved in water (0.5 ml), and this solution was added to the latex emulsion. A saturated solution of ammonium carbonate ( 5 ml, containing 2 drops of concentrated ammonium hydroxide) was added, and after stirring the mixture was dried in a microwave oven to a light foam.

### Example 7

Acrysol® ASE-75 (5 g, 2 g solids) was mixed with a solution of sucrose (4 g) in water (8 g). Aluminum/zirconium lactate (AAL) (0.36 g) was added, and then a saturated solution of ammonium carbonate (10 ml, containing 0.5 ml concentrated ammonium hydroxide) was added with stirring. This material was dried in a microwave oven to a soft, absorbent foam.

### Example 8

### Film

Sucrose (15 g) was mixed with water (15 ml) and then added to Acrysol® ASE-75 (17 g, 22% solids solution). Aluminum/zirconium lactate (AAL) (3.5 g of solution) was added, and after thorough mixing, ammonium hydroxide was added (4 ml, 28% concentration), and the resulting thick solution was thoroughly mixed and spread in a thin film and dried in a convection oven. After cooling and exposure to ambient humidity, the product was a soft and flexible film.

### Example 9

### Plaque

Sucrose (30 g) was dissolved in water (20 ml) and then added to Acrysol® ASE-75 emulsion (34 g of 22% solids solution). Aluminum/zirconium acetate (LAA) (7.3 g of solution) was added, and then just enough ammonium hydroxide was added to break the emulsion and form a gel. The gel was placed between two pieces of silicon-coated paper, flattened into a thick plaque, and dried in an oven for one and a half hours. The product was a thick rigid plaque that on exposured to high humidity became soft and flexible.

### Example 10

### Foam

Sucrose (10 g) was added to Acrysol® ASE-75 emulsion (25 g, 40% solids). Aluminum/zirconium acetate (LAA) (2.35 g of solution) was added, then solid ammonium carbonate (6.7 g) was added. The resulting foam was mixed and then dried in an oven for thirty minutes to provide an absorbent foam. The foam is stiff and brittle when removed from the oven but becomes soft and flexible when exposed to high humidity

### Example 11

### Foam

Sucrose (15 g) was mixed with Acrysol® ASE-75 emulsion (17 g, 22% solids). Ammonium zirconium carbonate (4.9 g of solution) was added. This induced the formation of a thick foam. The foam was placed on silicon-coated paper and dried in an oven. The resulting foam was soft and absorbant after exposure to ambient humidity.

## Claims

1. A wound dressing formed by drying a water-swellable, water-insoluble absorbent composition comprising an alkali soluble polyelectrolyte in solution with a polyhydroxylated material to which is added a polyvalent metal ion crosslinker.

2. A wound dressing according to claim 1 in which the alkali soluble polyelectrolyte is a synthetic carboxylated or sulfonated polymer.

3. A wound dressing according to claim 1 or claim 2 wherein the synthetic carboxylated or sulfonated polymer is polyacrylic acid or a partially hydrolyzed polyacrylamide.

4. A wound dressing according to any one of the preceding claims wherein the polyhydroxylate is a mono- di-, or oligosaccharide.

5. A wound dressing according to any of the preceding claims which contains from 10 to 80% by weight of polyhydroxylated material.

6. A wound dressing according to any one of the preceding claims wherein the metal ion crosslinker is a quadrivalent or a combination quadrivalent/trivalent metal ion crosslinker.

7. A wound dressing according to any one of the preceding claims wherein the metal ion crosslinker is zirconium ion or a mixture of zirconium and aluminum ions.

8. A wound dressing as claimed in any one of claims 1 to 7 composed of an emulsion of polyacrylic acid, mixed with at least 50% by weight of sucrose and crosslinked with zirconium ion or a mixture of aluminum/zirconium ions and dried.

9. The wound dressing according to any one of the preceding claims in the form of a film, a plaque or a cellular foam.

10. A water-swellable, water-insoluble superabsorbent composition comprising an alkali soluble polyelectrolyte admixed with a polyhydroxylated material which is crosslinked by means of a polyvalent metal ion crosslinker and neutralized by addition of a base to provide an absorbent polymer wherein the polyhydroxylated material is immobilized in said polymer.

11. A superabsorbent composition according to claim 10 which contains up to 90% by weight of alkali soluble polyelectrolyte.

12. A superabsorbent composition according to claim 10 or claim 11 wherein the alkali soluble polyelectrolyte is a polycarboxylated or polysulfonated polymer.

13. A superabsorbent composition according to any one of claims 10 to 12 wherein the alkali soluble polyelectrolyte is polyacrylic acid or a polyacrylate.

14. A superabsorbent composition according to any one of claims 10 to 13 which contains from 10 to 80% by weight of polyhydroxylate.

15. A superabsorbent composition according to any one of claims 10 to 14 which contains from 50 to 80% by weight of polyhydroxylate.

16. A superabsorbent composition according to any one of claims 10 to 15 wherein the polyhydroxylate is a mono-, di-, or oligosaccharide.

17. A superabsorbent composition according to any one of claims 10 to 16 wherein the metal ion crosslinker is zirconium or a mixture of zirconium and aluminum.

18. A method for preparing a superabsorbent containing high concentrations of polyhydroxylated material which comprises mixing a polyhydroxylated material with a solution or emulsion of a polyelectrolyte, adding a volatile neutralizing compound and a polyvalent metal ion crosslinker and drying said composition.

19. The method of claim 18 wherein the polyelectrolyte comprises up to 90% solids.

20. The method of claim 18 or claim 19 wherein said polyelectrolyte is polyacrylic acid or a polyacrylate.

21. The method of any one of claims 18 to 20 wherein the polyhydroxylated material is a mono-, di-, or oligosaccharide or soluble starch or polysaccharide.

22. The method of any one of claims 18 to 21 wherein the metal ion crosslinker is zirconium or a mixture of zirconium and aluminum.

23. The method of any one of Claims 18 to 22 wherein the polyhydroxylate/polyelectrolyte mixture is adjusted to a pH of approximately 7 by addition of a volatile base to which a metal ion crosslinker is added.

24. The method of any one of claims 18 to 23 wherein the polyhydroxylate/polyelectrolyte mixture is adjusted to a pH of approximately 7 by the addition of ammonium hydroxide and/or ammonium zirconium carbonate or mixtures thereof to the mixture.

## Patentansprüche

1. Wundverband aus einer trocknenden, wasserquellbaren, wasserunlöslichen, absorbierenden Zusammensetzung, die einen alkalilöslichen Polyelektrolyten in Losung mit einem polyhydroxylierten Material enthält, der ein mehrwertiges Metallion als Vernetzungsmittel zugesetzt ist.

2. Wundverband gemäß Anspruch 1, in dem der alkalilösliche Polyelektrolyte ein synthetisches carboxyliertes oder sulfoniertes Polymer ist.

3. Wundverband nach Anspruch 1 oder 2, bei dem das synthetische carboxylierte oder sulfonierte Polymer Polyacrylsäure oder ein teilweise hydrolisiertes Polyacrylamid ist.

4. Wundverband nach einem der vorstehenden Ansprüche, bei dem das Polyhydroxylat ein Mono-, Di- oder Oligosaccharid ist.

5. Wundverband nach einem der vorstehenden Ansprüche, der 10 bis 80 Gew.-% an polyhydroxyliertem Material enthält.

6. Wundverband nach einem der vorstehenden Ansprüche, bei dem das Metallion als Vernetzungsmittel ein vierwertiges oder eine Kombination aus vier- und dreiwertigen Metallionen als Vernetzungsmittel ist.

7. Wundverband nach einem der vorstehenden Ansprüche, bei dem das Metallion als Vernetzungsmittel ein Zirkoniumion oder ein Gemisch aus Zirkonium- und Aluminiumionen ist.

8. Wundverband nach einem der Ansprüche 1 bis 7 aus einer Polyacrylsäure-Emulsion, die mit mindestens 50 Gew.-% Saccharose gemischt und mit Zirkoniumionen oder einem Gemisch aus Zirkonium- und Aluminiumionen quervernetzt ist.

9. Wundverband nach einem der vorstehenden Ansprüche in Form eines Films, eines Plättchens oder eines zellulären Schaums.

10. Wasserquellbare, wasserlösliche, superabsorbierende Zusammensetzung, die im Gemisch mit einem polyhydroxylierten Material einen alkalilöslichen Polyelektrolyten enthält, der mit Hilfe eines polyvalenten Metallions als Vernetzungsmittel quervernetzt ist und durch Zugabe einer Base neutralisiert wurde, um ein absorbierendes Polymer zur Verfügung zu stellen, in dem das polyhydroxylierte Material im Polymer immobilisiert ist.

11. Superabsorbierende Zusammensetzung nach Anspruch 10, die bis zu 90 Gew.-% an alkalilöslichem Polyelektrolyten enthält.

12. Superabsorbierende Zusammensetzung nach Anspruch 10 oder 11, in welcher der alkalilösliche Polyelektrolyt ein polycarboxyliertes oder polysulfoniertes Polymer ist.

13. Superabsorbierende Zusammensetzung nach einem der Ansprüche 10 bis 12, in welcher der alkalilösliche Polyelektrolyt Polyacrylsäure oder ein Polyacrylat ist.

14. Superabsorbierende Zusammensetzung nach einem der Ansprüche 10 bis 13, die von 10 bis 80 Gew.-% an Polyhydroxylat enthält.

15. Superabsorbierende Zusammensetzung nach einem der Ansprüche 10 bis 14, die von 50 bis 80 Gew.-% an Polyhydroxylat enthält.

16. Superabsorbierende Zusammensetzung nach einem der Ansprüche 10 bis 15, in der das Polyhydroxylat ein Mono-, Di- oder Oligosaccharid ist.

17. Superabsorbierende Zusammensetzung nach einem der Ansprüche 10 bis 16, in der das Metallion als Vernetzungsmittel ein Zirkoniumion oder ein Gemisch aus Zirkoniumionen und Aluminiumionen darstellt.

18. Verfahren zur Herstellung eines superabsorbierenden Materials, welches hohe Konzentrationen eines polyhydroxylierten Materials enthält, bei dem man ein polyhydroxyliertes Material mit einem Lösung oder Emulsion eines Polyelektrolyten mischt, eine flüchtige, neutralisierende Verbindung und ein polyvalentes Metallion als Vernetzungsmittel zusetzt und die Zusamensetzung trocknet.

19. Verfahren nach Anspruch 18, bei dem der Polyelektrolyt bis zu 90 Gew.-% Feststoffe enthält.

20. Verfahren gemäß einem der Anspruch 18 oder 19, bei dem der Polyelektrolyt Polyacrylsäure oder ein Polyacrylat ist.

21. Verfahren gemäß einem der Ansprüche 18 bis 20, bei dem das polyhydroxylierte Material ein Mono-, Di- oder Oligosaccharid oder lösliche Stärke oder ein Polysaccharid ist.

22. Verfahren gemäß einem der Ansprüche 18 bis 21, bei dem das Metallion als Vernetzungsmittel ein Zirkoniumion oder ein Gemisch aus Zirkoniumionen und Aluminiumionen darstellt.

23. Verfahren gemäß einem der Ansprüche 18 bis 22, bei dem das Gemisch aus Polyhydroxylat und Polyelektrolyt durch Zugabe einer flüchtigen Base auf einen pH von etwa 7 eingestellt wird, zu dem ein Metallion als Vernetzungsmittel zugegeben wird.

24. Verfahren nach einem der Ansprüche 18 bis 23, bei dem das Gemisch aus Polyhydroxylat und Polyelektrolyt durch Zugabe von Ammoniumhydroxid und/oder Ammoniumzirkoniumcarboxylat oder Gemischen derselben zu dem Gemisch auf einen pH von etwa 7 eingestellt wird.

## Revendications

1. Pansement pour plaies formé par séchage d'une composition absorbante insoluble dans l'eau, pouvant gonfler dans l'eau comprenant un polyélectrolyte soluble dans un alcali en solution avec un matériau polyhydroxylé auquel est ajouté un agent de réticulation de type ion métallique polyvalent.

2. Pansement pour plaies suivant la revendication 1, dans lequel le polyélectrolyte soluble dans un alcali est un polymère carboxylé ou sulfoné de synthèse.

3. Pansement pour plaies suivant les revendications 1 ou 2, dans lequel le polymère carboxylé ou sulfoné de synthèse est un acide polyacrylique ou un polyacrylamide partiellement hydrolysé.

4. Pansement pour plaies suivant l'une quelconque des revendications précédentes, dans lequel le polyhydroxylat est un mono-, di- ou oligosaccharide.

5. Pansement pour plaies suivant l'une quelconque des revendications précédentes, qui contient de 10 à 80% en poids de matériau polyhydroxylé.

6. Pansement pour plaies suivant l'une quelconque des revendications précédentes, dans lequel l'agent de réticulation de type ion métallique est un agent de réticulation de type ion métallique quadrivalent ou une combinaison d'ions métalliques quadrivalent et trivalent.

7. Pansement pour plaies suivant l'une quelconque des revendications précédentes, dans lequel l'agent de réticulation de type ion métallique est l'ion zirconium ou un mélange d'ions zirconium et alumnium.

8. Pansement pour plaies suivant l'une quelconque des revendications 1 à 7, composé d'une émulsion d'acide polyacrylique, mélangée avec au moins 50% en poids de sucrose et réticulée avec l'ion zirconium ou un mélange d'ions aluminium et zirconium, et séchée.

9. Pansement pour plaies suivant l'une quelconque des revendications précédentes, sous la forme d'un film, d'une plaque ou d'une mousse cellulaire.

10. Composition superabsorbante insoluble dans l'eau, pouvant gonfler dans l'eau comprenant un polyélectrolyte soluble dans un alcali mélangé avec un matériau polyhydroxylé qui est réticulé au moyen d'un agent de réticulation de type ion métallique polyvalent et neutralisé par addition d'une base pour fournir un polymère absorbant, dans laquelle le matériau polyhydroxylé est immobilisé dans ce polymère.

11. Composition superabsorbante suivant la revendication 10, qui contient jusqu'à 90% en poids de polyélectrolyte soluble dans un alcali.

12. Composition superabsorbante suivant les revendications 10 ou 11, dans laquelle le polyélectrolyte soluble dans un alcali est un polymère polycarboxylé ou polysulfoné.

13. Composition superabsorbante suivant l'une quelconque des revendications 10 à 12, dans laquelle le polyélectrolyte soluble dans un alcali est un acide polyacrylique ou un polyacrylate.

14. Composition superabsorbante suivant l'une quelconque des revendications 10 à 13, qui contient de 10 à 80% en poids de polyhydroxylat.

15. Composition superabsorbante suivant l'une quelconque des revendications 10 à 14, qui contient de 50 à 80% en poids de polyhydroxylat.

16. Composition superabsorbante suivant l'une quelconque des revendications 10 à 15, dans laquelle le polyhydroxylat est un mono-, di- ou oligosaccharide.

17. Composition superabsorbante suivant l'une quelconque des revendications 10 à 16, dans laquelle l'agent de réticulation de type ion métallique est le zirconium ou un mélange de zirconium et d'aluminium.

18. Procédé pour la préparation d'un superabsorbant contenant de hautes concentrations de matériau polyhydroxylé, qui comprend le mélange d'un matériau polyhydroxylé avec une solution ou une émulsion d'un polyélectrolyte, l'addition d'un composé neutralisant volatil et d'un agent de réticulation de type ion métallique polyvalent et le séchage de cette composition.

19. Procédé suivant la revendication 18, dans lequel le polyélectrolyte comprend jusqu'à 90% de solides.

20. Procédé suivant l'une quelconque des revendications 18 ou 19, dans lequel ce polyélectrolyte est un acide polyacrylique ou un polyacrylate.

21. Procédé suivant l'une quelconque des revendications 18 à 20, dans lequel le matériau polyhydroxylé est un mono-, di- ou oligosaccharide ou un amidon soluble ou un polysaccharide.

22. Procédé suivant l'une quelconque des revendications 18 à 21, dans lequel l'agent de réticulation de type ion métallique est le zirconium ou un mélange de zirconium et d'aluminium.

23. Procédé suivant l'une quelconque des revendications 18 à 22, dans lequel le mélange de polyhydroxylat et de polyélectrolyte est ajusté à un pH d'environ 7 par addition d'une base volatile et additionné d'un agent de réticulation de type ion métallique.

24. Procédé suivant l'une quelconque des revendications 18 à 23, dans lequel le mélange de polyhydroxylat et de polyélectrolyte est ajusté à un pH d'environ 7 par addition au mélange d'hydroxyde d'ammonium et/ou de carbonate d'ammonium et de zirconium ou de leurs mélanges.
